# EUROPEAN PATENT APPLICATION

(11) **EP 2 842 949 A1**
(43) Date of publication of application: **04.03.2015**
(21) Application number: 13306193.7
(22) Date of filing: 02.09.2013
(51) Int. Cl.: C07D 257/10, H01G 9/20, H01L 31/00, A61K 31/395, A61P 35/00, A61N 5/06

(54) **Analogues of porphyrins, their method of preparation and use thereof especially in dye-sensitive solar cells**

(71) Applicant: Université d'Aix-Marseille, 13007 Marseille (FR); Centre National de la Recherche Scientifique (CNRS), 75016 Paris (FR); UNIVERSITE DE NANTES, 44000 Nantes (FR)
(72) Inventor: The designation of the inventor has not yet been filed
(74) Representative: Colombet, Alain André

(57) **Abstract**

The present invention relates to compounds of formula (I), their method of preparation and use thereof in particular in the fields of optoelectronic, photonic, photovoltaics and biology.

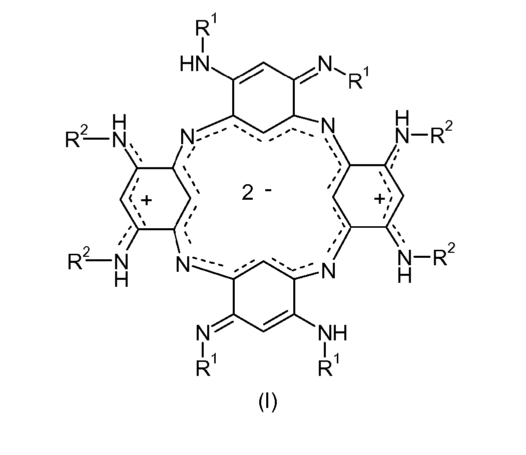

## Description

The present invention concerns pyrrol-free analogues of porphyrins, their method of preparation and their use thereof especially in the fields of photonics, organic electronics including photovoltaics, optoelectronics and biology including photodynamic therapies.

π-conjugated organic molecules (cyclic and/or linear) are widely used in the field of photonics, organic electronics including photovoltaics, optoelectronics and biology due to their geometrical and electronic structures which give them unique properties. The optic and electronic properties of those π-conjugated organic molecules are linked to a parameter named HOMO-LUMO gap (Eg) which corresponds to the energy difference between the Highest Occupied Molecular Orbital (HOMO) and the Lowest Unoccupied Molecular Orbital (LUMO). This gap should be as low as possible for the above-mentioned applications.

The molecules presenting a low Eg gap are often molecules of high dimensions. However, those molecules are weakly soluble which could be a huge limitation for the above mentioned applications. It is thus important to find new π-conjugated molecules that could present a low Eg gap and which could be soluble in organic medium and water.

Photodynamic therapy (PDT) is a form of phototherapy using nontoxic light-sensitive compounds that are exposed selectively to light, whereupon they becomes toxic to targeted malignant and other diseased cells, and thus can be used in the treatment of cancer for example. Most modern PDT applications involve three key components: a photosensitizer, a light source and oxygen. The wavelength of the light source needs to be appropriate for exciting the photosensitizer to produce reactive oxygen species. The combination of these three components leads to the chemical destruction of any tissues which have either selectively taken up the photosensitizer or have been locally exposed to light. This method has some disadvantages since only cancers accessible to light can be treated such as cancers near the surface of the skin (for example red light only has a penetration of about 1cm in living tissues). In order to treat other cancers which require a higher penetration into the tissues, the photosensitizer used should absorb in the near infrared region (NIR region) as those radiations penetrate more deeply in the skin.

Regarding photovoltaic technology, about 50% of the solar energy is in the near infrared region. Thus one of the major limiting factors for organic solar cells is the gap between the spectral absorption of the active layer and the solar emitting spectrum. The organic devices actually use various molecules in order to absorb the major part of the solar energy (from visible to near infrared). The use of a unique organic molecule absorbing on a large scale would enable an improvement in yield and costs. Organic solar cells, are based on a blend or an electron donor and hole transporting material (such as polythiophene) mixed with an electron acceptor and electronic conductor material (generally fullerene derivatives). Most of the photovoltaic cells actually used are based on silicon. However, this material is expensive and difficult to recycle. Dye-photosensitized solar cells also called Grätzel (or Graetzel) cells have been developed based on sensitized inorganic/organic hybride semi-conductors instead of silicon. In Grätzel cell, upon photo absorption, the dye injects an electron in the conduction band of the semi-conductor. These cells especially implement less expensive material, simple and low cost production techniques. Modules obtained are semi-flexible and semi-transparent which also open larger fields of application. There is thus a need to develop dyes for this type of solar cells with improved photoconversion efficiency.

Among the π-conjugated molecules, porphyrins are probably the most important and adaptable macrocycles. The major research activity on porphyrins covers a broadest area ranging from chemistry, materials science, physics, biology, engineering and medicine. Porphyrins are highly conjugated heterocyclic macrocycles composed of four pyrrol subunits interconnected via one-atom bridges forming a 16-membered central ring. These porphyrins present 18 delocalized π-electrons. However, these porphyrins do not absorb in the NIR region.

Also known from Muranaka et al (JACS, 2012, 134, 190-193) are analogues of hemiporphyrazine which absorb in the NIR region. However, these compounds are not versatile since their modifications are very limited. Indeed, it should be very useful to modify the macrocyclic molecule in order to tune the Eg gap, the control the solubility, the geometry, etc...

As a consequence there is a need to provide new macrocyclic molecules with properties that can be easily tailored upon chemical modifications (i.e. high versatility), and that have small dimensions, low Eg gap and absorbance in the NIR region.

The objective of the present invention is to provide aromatic macrocycles of small dimensions, preferably high symmetry and which are stable over time in various media.

Another objective of the present invention is to provide such macrocycles easily accessible (i.e. easy chemical engineering which allows high versatility).

Another objective is also to provide such macrocycles which can absorb light in a large spectral range and more specifically in the NIR region.

Another objective of the present invention is also to provide macrocycles which can be used in solar cells, such as in organic solar cells and particularly as dye for dye-sensitized solar cells (Grätzel cells).

Other objectives should appear by reading the below description of the invention.

All these objectives are met by the compounds according to the invention which comprise a central 16-membered ring bearing four nitrogen atoms and 18 π-electron and four 6-membered ring subunits.

The present invention relates to compound of formula (I): 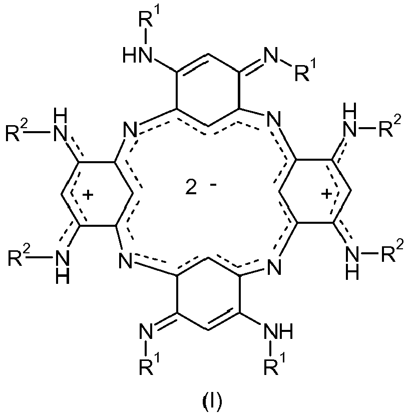 in which :
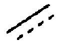 represents a single bond or a double bond provided that the central ring comprising 16 members (4 nitrogen atom and 12 carbon atoms) is an aromatic ring;
   each R¹ and R² are identical or different and represent:
   - a hydrogen atom;
   - an aliphatic chain, linear or branched, comprising from 1 to 30 carbon atoms;
   - a group of formula -(L¹)ₙ-A-(R)ᵣ;
with the proviso that at least one of R¹ and/or of R² is different from H;

A represents a cycloalkyl, heterocycloalkyl, an aryl or heteroaryl group, monocyclic or polycyclic; or an aliphatic chain, linear or branched, comprising from 1 to 30 carbon atoms;

r is an integer and represents from 0 to 5, for example 0, 1 or 2;

R, each identical or different represents:
- COR³,
- OR⁴ ,
- PO₃H₂,
- an alkyl chain, linear or branched comprising from 1 to 30 carbon atoms optionally substituted by at least one of (=O), OR⁴, COR³ and/or PO₃H₂;
- an aryl or heteroaryl optionally mono or polysubstituted by COR³, OR⁴, PO₃H₂, an alkyl chain, linear or branched comprising from 1 to 30 carbon atoms optionally substituted by at least one of (=O), OR⁴, COR³ and/or PO₃H₂;
R³ represents hydrogen, OR⁴, a linear or branched alkyl comprising from 1 to 30 carbon atoms and optionally substituted by at least one group (=O) and/or OR⁴ group;
R⁴ represents H, a linear or branched alkyl comprising from 1 to 30 carbon atoms;
n represents 0 or 1;
each L¹, identical or different, represents a linear or branched alkenyl or alkynyl comprising from 2 to 30 carbon atoms and comprising at least one double bond and/or at least one triple bond, preferably at least one of the double or triple bond is conjugated with the double bonds of the central ring and optionally with the double bond of A and optionally the double or triple bonds of L¹ are conjugated with each other.

Preferably, in the compound of formula (I), A represents an aryl, an heteroaryl or an aliphatic chain, linear or branched, comprising from 1 to 30 carbon atoms, preferably A represents aryl, preferably phenyl, or an aliphatic chain, linear or branched, comprising from 1 to 30 carbon atoms.

Preferably, in the compound of formula (I), at least one of R¹ and/or R² is -(L¹)ₙ-A-(R)ᵣ and R, identical or different represents:
- COOH,
- OH, preferably when A is phenyl there are at least two OH groups in ortho position;
- PO₃H₂,
- a C(O)CH₂C(O)Alk group, a C(O)CH(Alk)C(O)Alk group, a C(O)CH₂C(O)H group, a C(O)CH(Alk)C(O)H; wherein Alk is a linear or branched alkyl group comprising from 1 to 30 carbon atoms, preferably from 1 to 5 carbon atoms;
- an aryl substituted by a COOH or two OH groups in ortho position one relative to the other, a PO₃H₂ group, a C(O)CH₂C(O)Alk group, a C(O)CH(Alk)C(O)Alk group, a C(O)CH₂C(O)H group, a C(O)CH(Alk)C(O)H; wherein Alk is a linear or branched alkyl group comprising from 1 to 30 carbon atoms, preferably from 1 to 5 carbon atoms.

Advantageously, the compounds of the present invention comprise in R¹ and/or R²: COOH groups, PO₃H₂ groups, C(O)CH₂C(O)Alk groups, C(O)CH(Alk)C(O)Alk groups, C(O)CH₂C(O)H groups, C(O)CH(Alk)C(O)H groups, two OH groups which are functions that enable to chelate a compound and especially a TiO₂ compounds in solar cells.

Preferably, in the compound of formula (I) n is 0, and A is chosen among alkyl comprising from 1 to 10 carbon atoms, preferably from 1 to 5 carbon atoms, aryl comprising from 6 to 10 carbon atoms (mono or polycyclic) or heteroaryl comprising from 5 to 10 members (mono or polycyclic). Preferably, A is phenyl.

In another embodiment of the present invention n is 1 and A is chosen among alkyl comprising from 1 to 10 carbon atoms, preferably from 1 to 5 carbon atoms, aryl comprising from 6 to 10 carbon atoms (mono or polycyclic) or heteroaryl comprising from 5 to 10 members (mono or polycyclic). Preferably, A is phenyl, and L¹ represents a linear or branched alkenyl or alkynyl comprising from 2 to 30 carbon atoms and comprising at least one double bond and/or at least one triple bond, the double or triple bond being conjugated with the double bond of the central ring of formula (I), with the double bonds of A and with the other double bonds or triple bonds of L¹ if L¹ comprises more than four carbon atoms.

In a specific embodiment, the present invention relates to compound of formula (I): 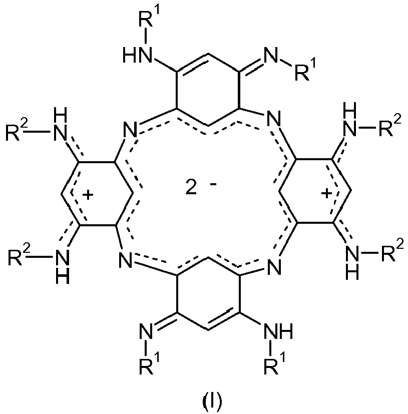 in which :
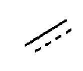 represents a single bond or a double bond provided that the central ring comprising 16 members (4 nitrogen atom and 12 carbon atoms) is an aromatic ring;
each R¹ and R² are identical or different and represent:
   - a hydrogen atom;
   - an aliphatic chain, linear or branched, comprising from 1 to 30 carbon atoms;
   - a group of formula -(L¹)ₙ-A-COR³; -(L¹)ₙ-A-PO₃H₂; or -A-(R⁵)_{q}
with the proviso that at least one of R¹ and/or of R² is different from H

A represents a cycloalkyl, heterocycloalkyl, aryl or heteroaryl group, monocyclic or polycyclic, each optionally mono- or poly-substituted for example by a linear or branched alkyl comprising from 1 to 30 carbon atoms and/or a group OR⁴; or an aliphatic chain, linear or branched, comprising from 1 to 30 carbon atoms;
R³ represents hydrogen, OR⁴, a linear or branched alkyl comprising from 1 to 30 carbon atoms and optionally substituted by at least one group (=O) and/or OR⁴;
R⁴ represents H, a linear or branched alkyl comprising from 1 to 30 carbon atoms;
q is an integer and represents from 1 to 5, for example 1 or 2;
R⁵, identical or different represents:
- OR⁴;
- an alkyl chain, linear or branched comprising from 1 to 30 carbon atoms optionally substituted by at least one of (=O), OR⁴, PO₃H₂, and/or COR³;
- aryl or heteroaryl optionally mono or polysubstituted by:
   ○ a linear or branched alkyl comprising from 1 to 30 carbon atoms optionally substituted by at least one (=O), OR⁴, PO₃H₂, and/or COR³;
   ○ a group OR⁴;
   ○ a group PO₃H₂; and/or
   ○ a group COR³;
n represents 0 or 1;
L¹ is as defined above.

In one embodiment of the present invention, A represents an aryl, an heteroaryl or an aliphatic chain, linear or branched, comprising from 1 to 30 carbon atoms, preferably A represents aryl, preferably phenyl, or an aliphatic chain, linear or branched, comprising from 1 to 30 carbon atoms.

Preferably, in the compounds of the invention, each R¹ and R² are identical or different and represent:
- a hydrogen atom;
- an aliphatic chain, linear or branched, comprising from 1 to 30 carbon atoms;
- a group of formula -(L¹)ₙ-A-COR³; -(L¹)ₙ-A-PO₃H₂; or -A-(R⁵)_{q}
with the proviso that at least one of R¹ and/or of R² is different from H and that at least one of R¹ and/or R² is -(L¹)ₙ-A-COR³; -(L¹)ₙ-A-PO₃H₂; or -A-(R⁵)_{q};
A represents a aryl or heteroaryl group, monocyclic or polycyclic, preferably aryl, for example phenyl, each optionally mono- or poly-substituted for example by a linear or branched alkyl comprising from 1 to 30 carbon atoms and/or a group OR⁴;
R³ represents hydrogen, OR⁴, a linear or branched alkyl comprising from 1 to 30 carbon atoms and optionally substituted by at least one group (=O) and/or OR⁴;
R⁴ represents H, a linear or branched alkyl comprising from 1 to 30 carbon atoms;
q is an integer and represents from 1 to 5, for example 1 or 2;
R⁵, identical or different represents:
- OR⁴;
- linear or branched alkyl comprising from 1 to 30 carbon atoms optionally substituted by at least one (=O), OR⁴, PO₃H₂, and/or COR³;
- aryl or heteroaryl optionally mono or polysubstituted by:
   ○ a linear or branched alkyl comprising from 1 to 30 carbon atoms optionally substituted by at least one (=O), OR⁴, PO₃H₂ and/or COR³;
   ○ a group OR⁴;
   ○ a group PO₃H₂; and/or
   ○ a group COR³;
   n represents 0 or 1;
   L¹ is as defined above.

Preferably, in the compounds of the invention, each R¹ and R² are identical or different and represent:
- a hydrogen atom;
- an aliphatic chain, linear or branched, comprising from 1 to 30 carbon atoms;
- a group of formula -(L¹)ₙ-A-COR³; -(L¹)ₙ-A-PO₃H₂; or -A-(R⁵)_{q;}
with the proviso that at least one of R¹ and/or of R² is different from H and that at least one of R¹ and/or R² is a group of formula -(L¹)ₙ-A-COR³; -(L¹)ₙ-A-PO₃H₂; or -A-(R⁵)_{q};
A represents an aliphatic chain, linear or branched, comprising from 1 to 30 carbon atoms;
R³ represents hydrogen, OR⁴, a linear or branched alkyl comprising from 1 to 30 carbon atoms and optionally substituted by at least one group (=O) and/or OR⁴;
R⁴ represents H, a linear or branched alkyl comprising from 1 to 30 carbon atoms;
R⁵ represents aryl or heteroaryl optionally substituted by at least a linear or branched alkyl comprising from 1 to 30 carbon atoms, a group OR⁴ and/or a group COR³;
q is an integer and represents from 1 to 5, for example 1 or 2;
R⁵, identical or different represents:
- OR⁴;
- aryl or heteroaryl optionally mono or polysubstituted by:
   ○ a linear or branched alkyl comprising from 1 to 30 carbon atoms optionally substituted by at least one group (=O), and/or OR⁴ group, a group PO₃H₂, a group COR³;
   ○ a group OR⁴;
   ○ a group PO₃H₂; and/or
   ○ a group COR³;
n represents 0 or 1;
L¹ is as defined above.

Preferably, in the compounds of the invention, each R¹ and R² are identical or different and represent:
- a hydrogen atom;
- an aliphatic chain, linear or branched, comprising from 1 to 30 carbon atoms
- a group of formula -(L¹)ₙ-A-COOR⁴; -(L¹)ₙ-A-CO-alk-CO-alk; -(L¹)ₙ-A-CO-alk-COH;-(L¹)ₙ-A-PO₃H₂; -A-aryl, the aryl being substituted by two OH group in ortho position one relative to the other and/or one CO group and one OH group in ortho position one relative to the other;
with the proviso that at least one of R¹ and/or of R² is different from H and at least one of R¹ and/or of R² is selected among a group of formula -(L¹)ₙ-A-COOR⁴; -(L¹)ₙ-A-CO-alk-CO-alk; -(L¹)ₙ-A-CO-alk-COH;-(L¹)ₙ-A-PO₃H₂; -A-aryl, the aryl being substituted by two OH group in ortho position one relative to the other and/or one CO group and one OH group in ortho position one relative to the other ;
n and L¹ being as defined above;
R⁴ represents H, a linear or branched alkyl comprising from 1 to 30 carbon atoms;
Alk represents a linear or branched alkyl comprising from 1 to 30 carbon atoms, preferably alk is CH₂;
A represents a cycloalkyl, heterocycloalkyl, aryl or heteroaryl group, monocyclic or polycyclic, preferably aryl, for example phenyl, each optionally mono- or poly-substituted for example by a linear or branched alkyl comprising from 1 to 30 carbon atoms and/or a group OR⁴; or an aliphatic chain, linear or branched, comprising from 1 to 30 carbon atoms.

Preferably, in the compounds of the invention, each R¹ and R² are identical or different and represent:
- a hydrogen atom;
- an aliphatic chain, linear or branched, comprising from 1 to 30 carbon atoms
- a group of formula -(L¹)ₙ-A-COOR⁴; -(L¹)ₙ-A-C(O)CH₂C(O)Alk, -(L¹)ₙ-A-C(O)CH(Alk)C(O)Alk, -(L¹)ₙ-A-C(O)CH₂C(O)H, -(L¹)ₙ-A-C(O)CH(Alk)C(O)H ; -(L¹)ₙ-A-PO₃H₂; -A-aryl, the aryl being substituted by two OH groups in ortho position one relative to the other and/or one CO group and one OH group in ortho position one relative to the other;
with the proviso that at least one of R¹ and/or of R² is different from H and at least one of R¹ and/or of R² is selected among a group of formula -(L¹)ₙ-A-COOR⁴; -(L¹)ₙ-A-C(O)CH₂C(O)Alk, -(L¹)ₙ-A-C(O)CH(Alk)C(O)Alk, -(L¹)ₙ-A-C(O)CH₂C(O)H, -(L¹)ₙ-A-C(O)CH(Alk)C(O)H ; -(L¹)ₙ-A-PO₃H₂; -A-aryl, the aryl being substituted by two OH group in ortho position one relative to the other and/or one CO group and one OH group in ortho position one relative to the other ;
n and L¹ being as defined above;
R⁴ represents H, a linear or branched alkyl comprising from 1 to 30 carbon atoms;
Alk represents a linear or branched alkyl comprising from 1 to 30 carbon atoms;
A represents an aryl or heteroaryl group, monocyclic or polycyclic, preferably aryl, for example phenyl, each optionally mono- or poly-substituted for example by a linear or branched alkyl comprising from 1 to 30 carbon atoms and/or a group OR⁴.

Preferably, in the compounds of the invention, each R¹ and R² are identical or different and represent:
- a hydrogen atom;
- an aliphatic chain, linear or branched, comprising from 1 to 30 carbon atoms;
- a group of formula -(L¹)ₙ-A-COOR⁴; -(L¹)ₙ-A-CO-alk-CO-alk; -(L¹)ₙ-A-C(O)CH₂C(O)Alk, -(L¹)ₙ-A-C(O)CH(Alk)C(O)Alk, -(L¹)ₙ-A-C(O)CH₂C(O)H, -(L¹)ₙ-A-C(O)CH(Alk)C(O)H-(L¹)ₙ-A-PO₃H₂; -A-aryl, the aryl being substituted two OH group in ortho position one relative to the other and/or one CO group and one OH group in ortho position one relative to the other;
with the proviso that at least one of R¹ and/or of R² is different from H and at least one of R¹ and/or of R² is selected among a group of formula -(L¹)ₙ-A-COOR⁴; -(L¹)ₙ-A-CO-alk-CO-alk; -(L¹)ₙ-A-C(O)CH₂C(O)Alk , -(L¹)ₙ-A-C(O)CH(Alk)C(O)Alk, -(L¹)ₙ-A-C(O)CH₂C(O)H, - (L¹)ₙ-A-C(O)CH(Alk)C(O)H-(L¹)ₙ-A-PO₃H₂; -A-aryl, the aryl being substituted two OH group in ortho position one relative to the other and/or one CO group and one OH group in ortho position one relative to the other;
n and L¹ being as defined above;
R⁴ represents H, a linear or branched alkyl comprising from 1 to 30 carbon atoms;
Alk represents a linear or branched alkyl comprising from 1 to 30 carbon atoms;
A represents an aliphatic chain, linear or branched, comprising from 1 to 30 carbon atoms.

Preferably, in the compounds of the invention, each R¹ and R² are identical or different and represent:
- a hydrogen atom;
- an aliphatic chain, linear or branched, comprising from 1 to 30 carbon atoms;
- a group of formula -(L¹)ₙ-A-COOH; -(L¹)ₙ-A-acetylacetonate; -(L¹)ₙ-A-PO₃H₂; -A-catechol;
with the proviso that at least one of R¹ and/or of R² is different from H and at least one of R¹ and/or of R² is selected among a group of formula -(L¹)ₙ-A-COOH; -(L¹)ₙ-A-acetylacetonate; -(L¹)ₙ-A-PO₃H₂; -A-catechol;
n and L¹ being as defined above;
A represents a cycloalkyl, heterocycloalkyl, aryl or heteroaryl group, monocyclic or polycyclic, preferably aryl, for example phenyl, each optionally mono- or poly-substituted for example by a linear or branched alkyl comprising from 1 to 30 carbon atoms and/or a group OR⁴; or an aliphatic chain, linear or branched, comprising from 1 to 30 carbon atoms.

Preferably, in the compounds of the invention, each R¹ and R² are identical or different and represent:
- a hydrogen atom;
- an aliphatic chain, linear or branched, comprising from 1 to 30 carbon atoms;
- a group of formula -(L¹)ₙ-A-COOH; -(L¹)ₙ-A-acetylacetonate; -(L¹)ₙ-A-PO₃H₂; -A-catechol;
with the proviso that at least one of R¹ and/or of R² is different from H and at least one of R¹ and/or of R² is selected among a group of formula -(L¹)ₙ-A-COOH; -(L¹)ₙ-A-acetylacetonate; -(L¹)ₙ-A-PO₃H₂; -A-catechol;
n and L¹ being as defined above;
A represents a aryl or heteroaryl group, preferably aryl, monocyclic or polycyclic, preferably aryl, for example phenyl, each optionally mono- or poly-substituted for example by a linear or branched alkyl comprising from 1 to 30 carbon atoms and/or a group OR⁴.

Preferably, in the compounds of the invention, each R¹ and R² are identical or different and represent:
- a hydrogen atom;
- an aliphatic chain, linear or branched, comprising from 1 to 30 carbon atoms;
- a group of formula -(L¹)ₙ-A-COOH; -(L¹)ₙ-A-acetylacetonate; -(L¹)ₙ-A-PO₃H₂; -A-catechol;
with the proviso that at least one of R¹ and/or of R² is different from H and at least one of R¹ and/or of R² is selected among a group of formula -(L¹)ₙ-A-COOH; -(L¹)ₙ-A-acetylacetonate; -(L¹)ₙ-A-PO₃H₂; -A-catechol;
n and L¹ being as defined above;
A represents an aliphatic chain, linear or branched, comprising from 1 to 30 carbon atoms.

In one embodiment in the compounds of the present invention n is 0, A is chosen among alkyl comprising from 1 to 10 carbon atoms, preferably from 1 to 5 carbon atoms, aryl comprising from 6 to 10 carbon atoms (mono or polycyclic) or heteroaryl comprising from 5 to 10 members (mono or polycyclic) wherein the aryl or heteroaryl is optionally mono- or poly-substituted for example by a linear or branched alkyl comprising from 1 to 30 carbon atoms and/or a group OR⁴. Preferably, A is phenyl optionally mono- or poly-substituted for example by a linear or branched alkyl comprising from 1 to 30 carbon atoms and/or a group OR⁴.

In another embodiment of the present invention n is 1 and A is chosen among alkyl comprising from 1 to 10 carbon atoms, preferably from 1 to 5 carbon atoms, aryl comprising from 6 to 10 carbon atoms (mono or polycyclic) or heteroaryl comprising from 5 to 10 members (mono or polycyclic) wherein the aryl or heteroaryl is optionally mono- or poly-substituted for example by a linear or branched alkyl comprising from 1 to 30 carbon atoms and/or a group OR⁴. Preferably, A is phenyl optionally mono- or poly-substituted for example by a linear or branched alkyl comprising from 1 to 30 carbon atoms and/or a group OR⁴, and L¹ represents a linear or branched alkenyl comprising from 2 to 30 carbon atoms and comprising at least one double bond and/or at least one triple bond, the double or triple bond being conjugated with the double bond of the central ring of formula (I), with the double bonds of A and with the other double bonds or triple bonds of L¹ if L¹ comprises more than four carbon atoms.

Preferably the compounds of the invention, each R1 and R2 are identical or different and represent:
- a hydrogen atom;
- an aliphatic chain, linear or branched, comprising from 1 to 30 carbon atoms;
- a group of formula - (L¹)ₙ-A-COOH wherein A represents an aliphatic chain, linear or branched, comprising from 1 to 30 carbon atoms, for example from 1 to 5 carbon atoms, an aryl or heteroaryl group, monocyclic or polycyclic, the monocycle or polycycle being optionally mono- or polysubstituted by for example a linear or branched alkyl comprising from 1 to 30 carbon atoms and/or a group OR⁴;
- with the proviso that at least one of R¹ and/or R² is different from H and at least one of R¹ and/or of R² is selected among a group of formula -(L¹)ₙ-A-COOH.

In another embodiment of the present invention each R¹ and R² are identical or different and represent:
- a hydrogen atom;
- an aliphatic chain, linear or branched, comprising from 1 to 30 carbon atoms;
- a group of formula --(L¹)ₙ-A¹-COOH wherein A¹ represents a, aryl or heteroaryl group, monocyclic or polycyclic, the monocycle or polycycle being optionally mono- or polysubstituted by for example by a linear or branched alkyl comprising from 1 to 30 carbon atoms and/or a group OR⁴;
with the proviso that at least one of R¹ and/or R² is different from H and at least one of R¹ and/or of R² is selected among a group of formula -(L¹)ₙ-A¹-COOH.

Preferably, in this embodiment A¹ is chosen among aryl comprising from 6 to 10 carbon atoms or heteroaryl comprising from 5 to 10 members and when n is 1, L¹ represents a linear or branched alkyl comprising from 2 to 30 carbon atoms and comprising at least one double bond and/or at least one triple bond, the double or triple bond being conjugated with the double bond of the central ring of formula (I) and with the double bonds of A and with the other double bonds or triple bonds of L¹ if L¹ comprises more than four carbon atoms. Preferably, A¹ is phenyl optionally substituted and when n is 1, L¹ represents a linear or branched alkyl comprising from 2 to 30 carbon atoms and comprising at least one double bond and/or at least one triple bond, the double or triple bond being conjugated with the double bond of the central ring of formula (I) and with the double bonds of A and with the other double bonds or triple bonds of L¹ if L¹ comprises more than four carbon atoms.

Preferably, in this embodiment n is 0.

In another embodiment of the present invention each R¹ and R² are identical or different and represent:
- a hydrogen atom;
- an aliphatic chain, linear or branched, comprising from 1 to 30 carbon atoms;
- a group of formula alkyl-COOH, wherein the alkyl is linear or branched and comprised from 1 to 30 carbon atoms, preferably from 1 to 10 carbon atoms, for example from 1 to 5 carbon atoms,
with the proviso that at least one of R¹ and/or R² is different from H and at least one of R¹ and/or of R² is selected among a group of formula alkyl-COOH.

Preferably, in the compounds according to the invention R² represents H and R¹ is as defined above in the different embodiments presented, with the proviso that at least one of R¹ is different from H and at least one of the R¹ is selected among a group of formula -(L¹)ₙ-A-COR³; -(L¹)ₙ-A-PO₃H₂; or -A-(R⁵)_{q}.

According to the present invention:
- aryl is an aromatic mono- or polycycle comprising from 6 to 10 carbon atoms, preferably phenyl;
- cycloalkyl is a mono- or polycycle comprising from 5 to 10 carbon atoms;
- heteroaryl is an aromatic mono- or polycycle comprising from 5 to 10 members including at least one heteroatom for example chosen among N, O, S;
- heterocycloalkyl is a mono- or polycycle comprising from 5 to 10 members including at least one heteroatom for example chosen among N, O, S.

It should be understood that compounds of formula (I) according to the invention are bis-zwitterionic compounds in which two positive charges and two negative charges are delocalized.

It should also be understood that 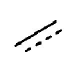 represents either a single bond or a double bond, which are delocalized in the cycle. Formula (I) comprises especially the following formula: 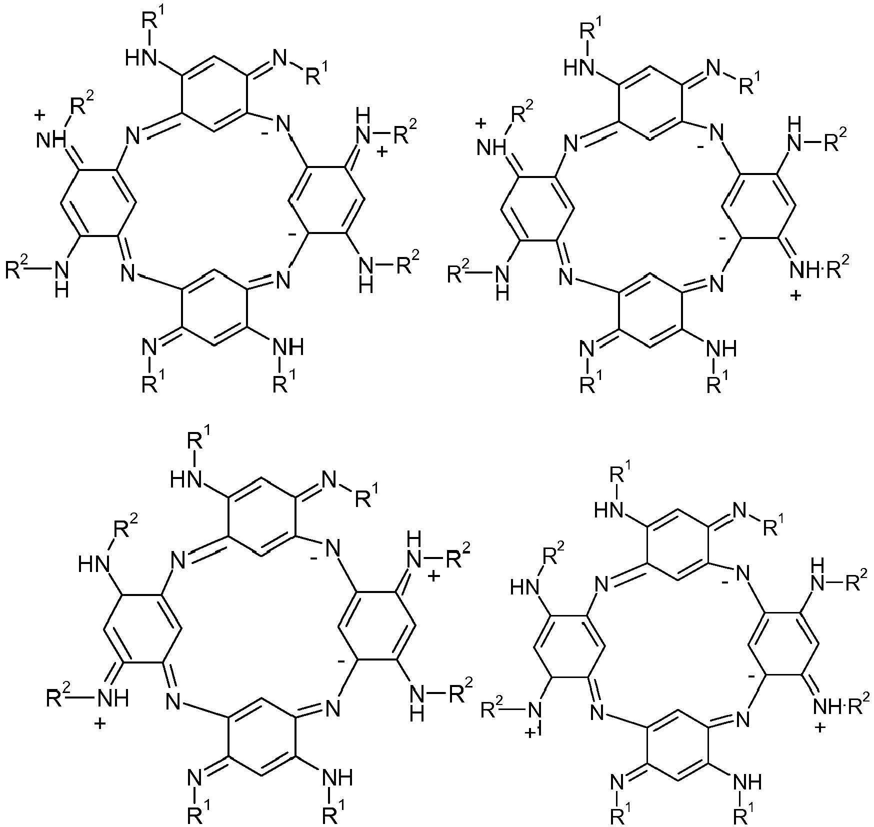 Those formulae show the delocalisation of the positive charge but it should be understood that the negative charge is also delocalisable in the central ring.

As mentioned above, the invention also relates to compounds of formula (I) in their ionic forms. Those compounds in ionic forms can be obtained by reacting the bis-zwitterionic compounds of formula (I) with at least one mole of an acid. The acid being preferably chosen among CF₃COOH or HX, X being an anion (halogen atom, BF₄⁻, PF₆⁻, ClO₄⁻ etc...). Those compounds in ionic form can be as follows: 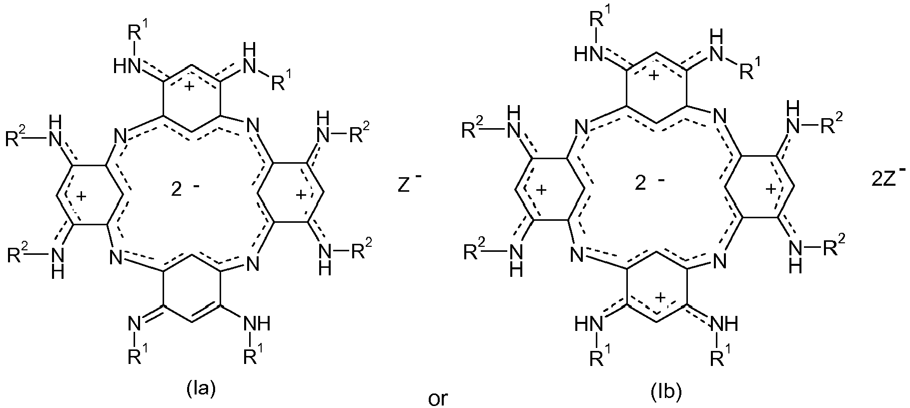 wherein Z represents an anion, for example X, BF₄, PF₆, ClO₄ or CF₃COO.
Preferably, the compounds in ionic form are of formula (Ib).

The compounds in ionic form can also be obtained by reacting the bis-zwitterionic compounds of formula (I) with at least one mole of a base. The base is preferably chosen among strong base, for example base chosen among NEt₃, NaOtBu, BuLi, NaOH.
Those compounds in ionic form can be as follows: 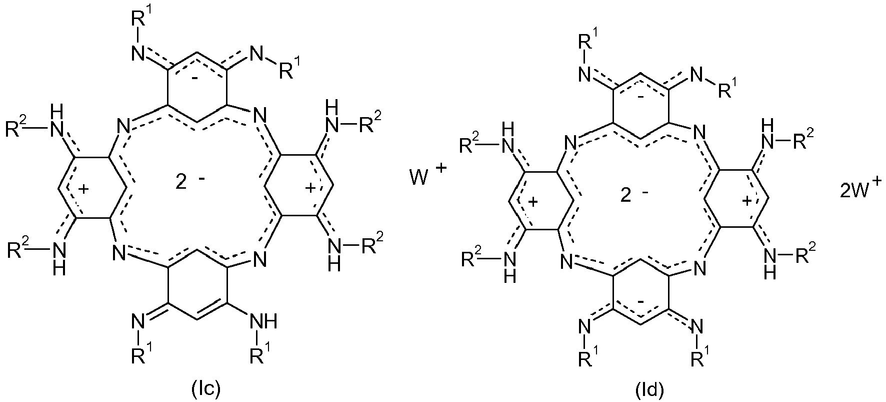 or 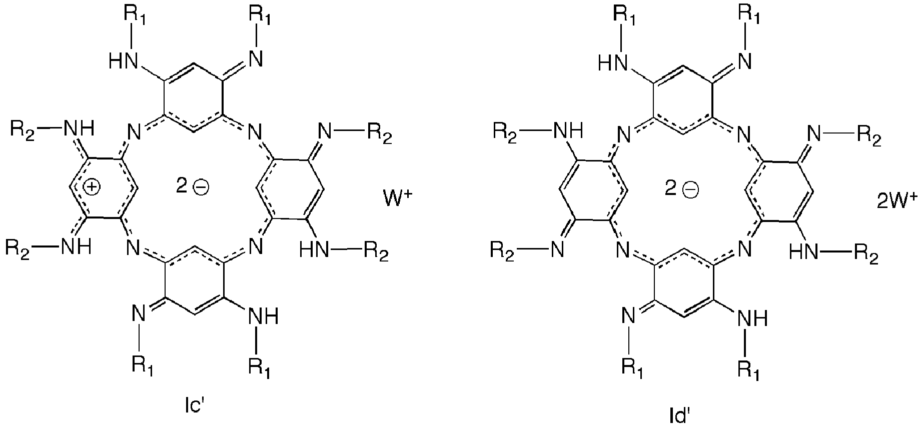 wherein W represents Na, NHEt₃, Li.

Preferably, the compounds in ionic form are of formula (Ic') et (Id').

As mentioned above, the invention also relates to compounds of formula (I) in the form of chelates since Density Functional Theory (DFT) calculation (as shown in the examples) revealed the possible stabilization of high oxidation state metals in the center (metal having a degree of oxidation of 4 which could link covalently with four carbon atoms of the central ring) and/or the metallation of the external parts (metal having a degree of oxidation of 2 which could be linked to the nitrogens at the periphery of the macrocycle. The metal is for example tetracoordinated, pentacoordinated or hexacoordinated. Those compounds in their chelate form can be as following (Ie)-(Ii): 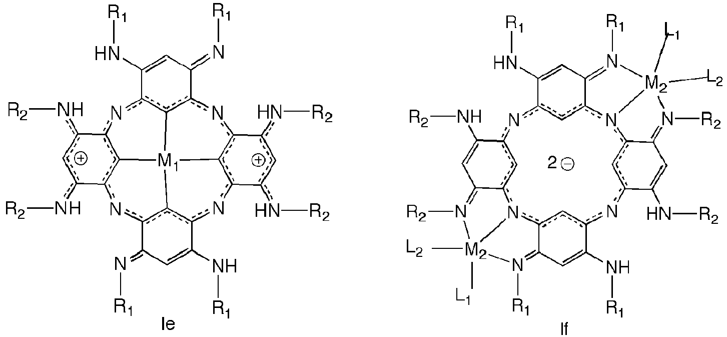 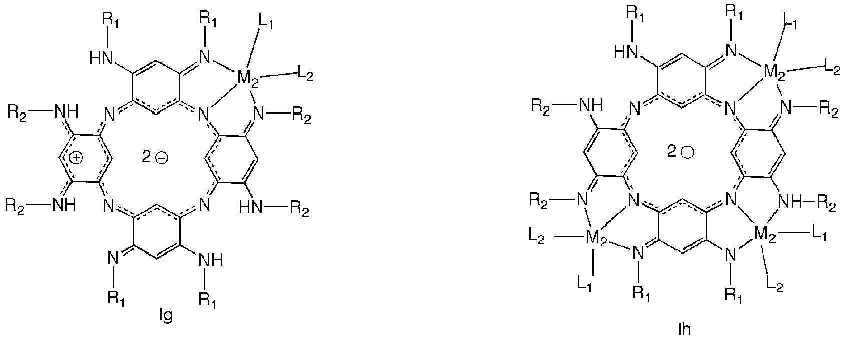 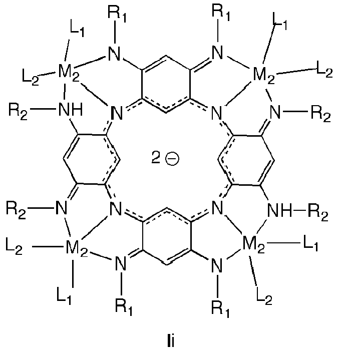 wherein:
R¹ and R² are defined as above,
M₁ is chosen among the transition metals having a degree of oxidation of 4 and M₂ is chosen among the transition metals having a degree of oxidation of 2 or lanthanides. M₁ is preferably chosen among vanadium, titanium, iridium and platinum. M₂ is preferably chosen among palladium, nickel, cobalt, zinc, iron, europium, cerium, gadolinium, lanthane;
L₁ and L₂, identical or different, is a monodentate, bidentate or tridentate ligand, preferably is a ligand which can give 1 covalent bond or 2 electrons, and/or may be optionally bound together. L₁ and L₂ can form together a group acac (L is O and the L and the two L are bound together), an amine, a phosphine, an aryl, or a halogen. It is also possible, in order to have tetracoordinate, pentacoordinate or hexacoordinate that the complex comprises m L, m being chosen among 2 to 6.

The chelation of those metals can enable to improve the Eg gap of the molecules and also to absorb in infrared.

According to the present invention, the terms below have the following meanings:
- an aromatic compound contains a set of covalently bound atoms with specific characteristics:

1. A delocalized conjugated π system, most commonly an arrangement of alternating single and double bonds
2. A structure with all the contributing atoms of the aromatic ring in the same plane
3. Contributing atoms arranged in one or more rings
4. A number of π delocalized electrons that is 4n' + 2, where n' is an integer from 0 to 4.
   In the present invention n' is 4;
   - a halogen atom corresponds to a fluorine, chlorine, bromine or iodine atom;
   - an alkyl group corresponds to a saturated, linear or branched aliphatic group having from 1 to 30 carbon atoms, preferably from 1 to 18 carbon atoms, for example from 1 to 10 carbon atoms.
   - a cycloalkyl group corresponds to a cyclic alkyl group comprising from 3 to 6 members. The following examples may be cited: cyclopropyl, cyclobutyl, cyclopentyl, cyclohexyl, etc;
   - a heterocycle group is preferably a 5 to 10-membered cycle comprising at least one heteroatom, for example 1, 2 or 3 heteroatoms, preferably chosen among N, S or O;
   - an heteroaryl group corresponds to an aromatic heterocycle comprising from 5 to 10 members, preferably 5 or 6 members, preferably including from 1 to 3 heteroatoms preferably chosen among N, S or O;
   - an aryl group corresponds to an aromatic cycloalkyl comprising from 6 to 10 members, preferably 6 members;
   - a transition metal is an element from group 3 to group 12 and period 4 to period 7, except Lr and Lu, of the periodic table of the element;
   - a lanthanide is an element of the periodic table having an atomic number from 57 through 71.

It is understood that (Rᵢ)ⱼ is such that for each of the j occurrence Rᵢ must be identical or different.

The compounds according to the present invention are surprisingly stable and could be exposed to air for months as a solid or for days in solution in DMSO without detectable changes. Without being bound to any theory, this high stability is not only due to aromatic behavior of the central ring but most likely to the zwitterionic character of the whole molecule.

Advantageously, the compounds of the invention absorb in all the visible, in UV and in the NIR region, especially between 300 and 1000 nm. Especially, the inventors have shown that the compounds of the invention absorb radiations around 880 nm which is in the NIR region. This specific range of absorbance especially enables to use those compounds in the field of electronics, photovoltaics, optoelectronics and photodynamic therapies.

As mentioned above, the range of absorption can advantageously be modified by modifying the group R¹ and R² and/or by chelating metals.

Advantageously, the compounds of the invention present a Eg gap comprised between 0.5 and 4 eV, preferably between 1 and 4 eV. For example, compound of formula (I) wherein R¹ and R² represent H present a Eg gap of 1.4 eV. This gap is determined by DFT calculations and by experiments (UV absorption) which is a technique well known by the skilled person. The Eg gap determined by electrochemical experiments is preferably of 1.01 eV.

Without being bound to any theory, the inventors have found that the weak HOMO-LUMO gap can be explained on one hand by the dianionic character of the central cycle and on the other hand by the similarity of the frontier molecular orbitals (HOMO and LUMO) which are totally delocalized. The value of this gap is important to determine the possible applications for the molecules. For example in order to be used in optoelectronics or photodynamic therapies, the gap has to be as low as possible.

The value of the gap should be modified by the modification of the group R¹ and/or R² making the compounds of formula (I) highly versatile. The value of the gap should also be modified by chelating compound of formula (I) with metal(s) as mentioned previously.

The compounds of the invention present some electronic properties similar to the compounds disclosed by Qian et al (Chem. Asian. J., 2010, 5, 1006).

As a consequence, in view of their specific structural and electronic properties (especially due to their NIR absorbing properties) as well as their specific versatility, the compounds according to the invention can be used in many fields. For examples, the compounds of the invention could be used in electronics, more specifically in optoelectronics; in the field of photovoltaics; in the field of photonics; in the field of biology, especially in the field of photodynamic therapies.

Especially, when used in photovoltaics, the compounds of the invention having low dimensions and a weak Eg gap have different advantages compared to the polymers actually used: there are easy to synthesize, to functionalize (there are versatile), to purify... Since they absorb in all visible and partly in the NIR range, the compounds of the invention can be used alone in the photovoltaic cells and there is no need of different molecules as mentioned above. The compounds of the invention can thus be used as components of solar cells, including organic molecular cells, polymer cells and dye-sensitized solar cells. In those cells the compounds of the inventions serve as charge-transport agents and/or absorbing agents.

As mentioned above, the compounds of the invention contain few atoms and absorb in the NIR region which make them good candidates for being used in photodynamic therapies for the treatment of cancer, including those in deep tissues. In photodynamic therapies, the compounds of the invention are used as photosensitizers. The compounds of the invention are very useful in such an application since they can be modified, by modifying the R¹ and R² group or by chelation, which enables to control the absorption range of the light as well as the solubility of the compounds.

The compounds of the invention could also be used as ligand in coordination chemistry since as mentioned above they can make chelate with metals.

The present invention also relates to the use of the compound of formula (I) in solar cells, particularly in organic solar cells and as dye in dye-sensitized solar cell such as Grätzel cell.

Indeed, due to their specific properties as mentioned above and especially the Eg gap and the properties of absorption of near IR, the compounds according to the invention are particularly useful as dye for dye-sensitized solar cell. Compounds of the invention where A is an aryl or heteroaryl, monocyclic or polycyclic, are particularly advantageous for the use in dye-sensitized cells especially due to the possible delocalisation of the charge after excitation of the compound by solar radiation.

According to the present invention dye-sensitized solar cell (Grätzel cell), intends to mean solar cells comprising two glass plaque defining an inner medium and one of them comprising a porous film of a semi-conductor, for example TiO₂, coated with a monolayer of a dye.

The present invention also relates to solar cell, the solar cell can be such as organic solar cells or dye-sensitized solar cells such as Grätzel cell, comprising a compound according to the invention. Preferably, the present invention relates to such as dye-sensitized solar cells, comprising two substrates defining an inner medium which comprises a semi-conductor, for example TiO₂, and a compound of the invention as a dye.

The present invention also relates to a process (P1) for the preparation of compound of formula (I) when at least one of R¹ or one of R² is different from H comprising the following steps:
a1) reacting a 1,5-Q₂-2,4-dinitrobenzene with 0,5 equivalent of tetraaminobenzene in the presence of a base, Q, identical or different being a leaving group;
b1) reacting the compound obtained in step a1) with one equivalent of a compound derived from tetraaminobenzene in which at least one and at most two of NH₂ group is substituted with a group R¹ or R² different from H, in the presence of a base;
c1) reduction of the compound obtained in step b1) in the presence of a reducing agent which under air is converted into compound of formula (I).

The present invention also relates to a process (P2) for the preparation of compound of formula (I) when at least one of R¹ or one of R² is different form H comprising the following steps:
a2) reacting a 1,5-Q₂-2,4-dinitrobenzene with 1 equivalent of a compound derived from tetraaminobenzene in which at least one and at most two of NH₂ group is substituted with a group R¹ or R² different from H, in the presence of a base, Q, identical or different being a leaving group;
b2) reduction of the compound obtained in step a2) in the presence of a reducing agent which under air is converted into compound of formula (I).

The present invention also relates to a process (P3) for the preparation of compound of formula (I) when at least one of R¹ or one of R² is different form H comprising the following steps:
a3) reacting a 1,5-Q₂-2,4-dinitrobenzene with 0,5 equivalent of a compound derived from tetraaminobenzene in which at least one and at most two of NH₂ group is substituted with a first group R¹ or R² different from H, in the presence of a base, Q, identical or different being a leaving group;
b3) reacting the compound obtained in step a3) with one equivalent of a compound derived from tetraaminobenzene in which at least one and at most two of NH₂ group is substituted with a second group R¹ or R² different from H and different from the one of step a3), in the presence of a base;
c3) reduction of the compound obtained in step b3) in the presence of a reducing agent which under air is converted into compound of formula (I).

Preferably, the reducing agent in process (P1), (P2) and (P3) is chosen among SnCl₂; H₂, Pd/C; hydrazine; ammonium formate (NH₄,HCO₂,Pd/C).

Preferably, the leaving group in process (P1), (P2) and (P3) is chosen among halides, triflates (OSO₂CF₃), sulfonate esters such as tosylate or mesylate. Preferably the leaving group is chosen among halides. Preferably 1,5-Q₂-2,4-dinitrobenzene is 1,5-difluoro-2,4-dinitrobenzene.

Preferably, steps a1), b1), a2), a3) and c3) are carried out in the presence of a solvent, for example acetonitrile.

Preferably steps a1), b1), a2), a3) and c3) are carried out at low temperature, for example between -10 and 10°C, especially at 0°C, then the temperature is raised to room temperature, then, finally the reaction is carried out under reflux.

Preferably, in steps a1), b1), a2), a3) and c3) the base is for example diisopropylethylamine (DIPEA).

Preferably, in steps c1), b2) and c3), the reducing agent is used in large excess.

Preferably, in step c1), b2) and c3) can be implemented in the presence of an acid preferably for example when the reducing agent is SnCl₂, the acid is preferably HX, preferably HCl.

Preferably, steps c1, b2 and c3) are carried out at a temperature of around 50-100°C, for example 70°C.

Compounds of formula (Ia) and (Ib) are obtained by reacting compounds of formula (I) with at least one mole of an acid.

Compounds of formula (Ic) and (Id) are obtained by reacting compounds of formula (I) with at least one mole of a base.

Compounds of formula (Ie) to (Ii) are obtained by mixing compounds of formula (I) in solution in a solvent with the metal in solution in a solvent.

Advantageously, the synthetic accessibility to compounds of the invention is straightforward and highly versatile, as substituents on the peripheric nitrogen atoms are easily introduced and can be easily varied for tuning the properties of the compounds (solubility, geometry, donor/acceptor properties...)

The following examples describe the synthesis of some compounds according to the invention. These examples are not intended to be limitative and only illustrate the present invention.

### Example 1: Preparation of compounds (1.1) and (1.2)

### Step a1) Synthesis of Intermediate (a1)

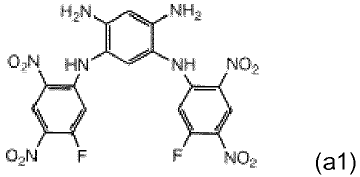

The commercially available 1,5-difluoro-2,4-dinitrobenzene was reacted with 0.5 equiv. of tetraaminobenzene in the presence of base (DIPEA) under inert atmosphere. The solution was stirred at 0°C for 14 h and the resulting solid was isolated by filtration and washed affording (a1).

### Step b1) Synthesis of Intermediate (b1.1 R1=C₆H₄COOH) and (b1.2 R1= CH₂COOH)

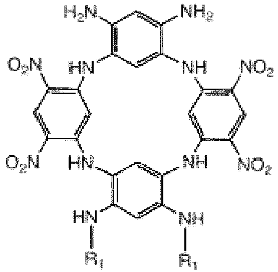

The triaryl derivative (a1) was reacted with 1 equiv. of disubstitued tetraaminobenzene - that could be prepared in two steps from 1,5-difluoro-2,4-dinitrobenzene and primary amines bearing COOH functions - in the presence of base (DIPEA) under inert atmosphere. The solution was stirred and the obtained solid was isolated by filtration and washed affording (b1.1) or (b1.2).

### Step c1) Synthesis of target (1.1 R1=C₆H₄COOH) or (1.2 R1= CH₂COOH)

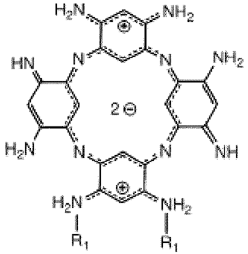

Tetranitro-tetraaminoazacalixphyrins (b1.1) or (b1.2) were then reacted in the presence of reducing agent under inert atmosphere affording the corresponding octaamino-azacalixphyrins - not isolated - which under air are converted into azacalixphyrins respectively (1.1) or (1.2).

### Example 2: Preparation of compounds (2.1) and (2.2)

### Step a2) Synthesis of Intermediate (a2.1 R1=C₆H₄COOH) or (a2.2 R1 = CH₂COOH)

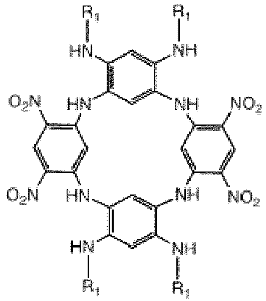

The commercially available 1,5-difluoro-2,4-dinitrobenzene was reacted with 1 equiv. of disubstitued tetraaminobenzene - that could be prepared in two steps from 1,5-difluoro-2,4-dinitrobenzene and primary amines bearing COOH functions - in the presence of base (DIPEA) under inert atmosphere. The solution was stirred and the obtained solid was isolated by filtration and washed affording (a2.1) or (a2.2).

### Steps b2) Synthesis of target (2.1 R1=C₆H₄COOH) or (2.2 R1= CH₂COOH)

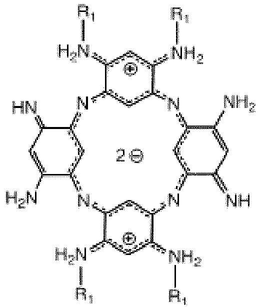

Tetranitro-tetraaminoazacalixphyrins (a2.1) or (a2.2) were then reacted in the presence of reducing agent under inert atmosphere affording the corresponding octaamino-azacalixphyrins - not isolated - which under air are converted into azacalixphyrins respectively (2.1) or (2.2).

### Example 3: Preparation of compounds (3a) and (3b)

### Step a3) Synthesis of Intermediate (a3.1 R1=C₆H₄COOH) or (a3.2 R1= CH₂COOH)

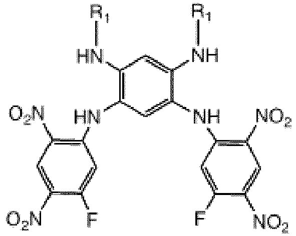

The commercially available 1,5-difluoro-2,4-dinitrobenzene was reacted with 0.5 equiv. of disubstitued tetraaminobenzene - that could be prepared in two steps from 1,5-difluoro-2,4-dinitrobenzene and primary amines bearing COOH functions - in the presence of base (DIPEA) under inert atmosphere. The solution was stirred and the crude product was purified by column chromatography (SiO₂) affording (a3.1) or (a3.2).

### Step b3) Synthesis of Intermediate (b3.1 R1=C₆H₄COOH, R2=nBu) or (b3.2 R1= CH₂COOH, R2=nBu)

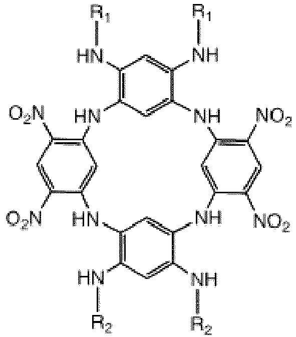

The triaryl derivatives (a3.1) or (a3.2) were reacted with 1 equiv. of dialkyltetraaminobenzene (5) - that could be prepared in two steps from 1,5-difluoro-2,4-dinitrobenzene and primary alkylamines - in the presence of base (DIPEA) under inert atmosphere. The solution was stirred and the crude product was purified by column chromatography (SiO₂) affording respectively (b3.1) or (b3.2).

### Step c3) Synthesis of target (3.1 R1=C₆H₄COOH, R2=nBu) or (3.2 R1= CH₂COOH, R2=nBu)

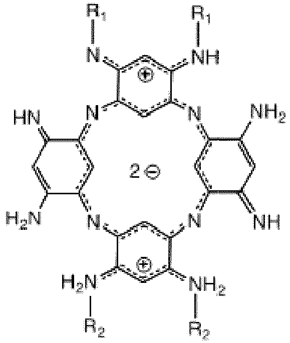

Tetranitro-azacalixphyrins (b3.1) or (b3.2) were then reacted in the presence of reducing agent under inert atmosphere affording the corresponding octaamino-azacalixphyrins - not isolated - which under air are converted into azacalixphyrins respectively (3.1) or (3.2).

### Example 4: Preparation and photovoltaic measurement of a Grätzel cell

FTO conductive glass substrates (F-doped SnO₂) were cleaned by successive sonication in soapy water, then an ethanolic solution of HCl (0.1 M) for 10 minutes, and finally dried in air. TiO₂ films were then prepared in three steps. A first treatment is applied by immersion for 30 min in an aqueous TiCl₄ solution at 80°C. Layers of TiO₂ were then screen printed with transparent colloidal paste DSL 18NR-T and light scattering DSL 18NR-AO (Dyesol) as final layer, with 20-minute long drying steps at 150°C between each layer. The obtained substrates were then sintered at 450°C, following a progressive heating ramp (325°C for 5 min, 375°C for 5 min, 450°C for 30 min). A second TiCl₄ treatment was immediately conducted afterwards. Thicknesses were measured by a Sloan Dektak 3 profilometer and are in the range of 12 µm. The prepared TiO₂ electrodes were soaked while still hot (80°C) in a 0.1 mM solution of the dye in a suitable solvent for 12 hours. Solar cells were prepared using the dye-sensitized electrodes as the working electrodes and platinum-coated conducting glass electrodes as counter electrodes. The latter were prepared by chemical deposition of platinum from hexachloroplatinic acid in distilled isopropanol (2 mg per mL) and subsequent firing at 380°C for 20 minutes. The two electrodes were placed on top of each other and sealed using a thin transparent film of Surlyn polymer (DuPont, 25 µm) as a spacer to form the electrolyte space. A drop of electrolyte was introduced by vacuum back filling through a predrilled hole in the counter electrode, and the photovoltaic device was sealed afterwards with surlyn and a cover glass. The cell had an active area of ca. 0.25 cm². The current-voltage characteristics were determined by applying an external potential bias to the cell and measuring the photocurrent using a Keithley model 2400 digital source meter. The overall conversion efficiency (η) of the photovoltaic cell is calculated from the integral photocurrent density (Jsc), the open-circuit photovoltage (Voc), the fill factor of the cell (FF), and the intensity of the incident light (IPh). The photovoltaic cell was illuminated with an Oriel lamp calibrated to AM 1.5 (air mass) intensity (1000 W.m⁻²).

## Claims

1. Compound of formula (I): 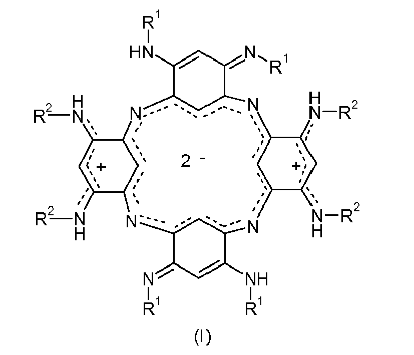 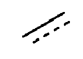 represents a single bond or a double bond provided that the central ring comprising 16 members (4 nitrogen atom and 12 carbon atoms) is an aromatic ring;
each R¹ and R² are identical or different and represent:
- a hydrogen atom;
- an aliphatic chain, linear or branched, comprising from 1 to 30 carbon atoms;
- a group of formula -(L¹)ₙ-A-(R)ᵣ;
with the proviso that at least one of R¹ and/or of R² is different from H;
A represents a cycloalkyl, heterocycloalkyl, an aryl or heteroaryl group, monocyclic or polycyclic; or an aliphatic chain, linear or branched, comprising from 1 to 30 carbon atoms;
r is an integer and represents from 0 to 5, for example 0, 1 or 2;
R, each identical or different represents:
- COR³,
- OR⁴,
- PO₃H₂,
- an alkyl chain, linear or branched comprising from 1 to 30 carbon atoms optionally substituted by at least one of (=O), OR⁴ group, COR³, and/or PO₃H₂;
- an aryl or heteroaryl optionally mono or polysubstituted by COR³, OR⁴, PO₃H₂, an alkyl chain, linear or branched comprising from 1 to 30 carbon atoms optionally substituted by at least one of (=O), OR⁴ group, COR³, and/or PO₃H₂;
R³ represents hydrogen, OR⁴, a linear or branched alkyl comprising from 1 to 30 carbon atoms and optionally substituted by at least one group (=O) and/or OR⁴ group;
R⁴ represents H, a linear or branched alkyl comprising from 1 to 30 carbon atoms;
n represents 0 or 1;
each L¹, identical or different, represents a linear or branched alkenyl or alkynyl comprising from 2 to 30 carbon atoms and comprising at least one double bond and/or at least one triple bond, preferably at least one of the double or triple bond is conjugated with the double bonds of the central ring and optionally with the double bond of A and optionally the double or triple bonds of L¹ are conjugated with each other.

2. Compound according to claim 1, in which A represents an aryl, an heteroaryl or an aliphatic chain, linear or branched, comprising from 1 to 30 carbon atoms, preferably A represents aryl, preferably phenyl, or an aliphatic chain, linear or branched, comprising from 1 to 30 carbon atoms.

3. Compound according to claim 1 or 2, in which at least one of R1 and/or R2 is -(L1)n-A-(R)r and R, identical or different represents:
- COOH,
- OH, preferably when A is phenyl there are at least two OH group in ortho position;
- PO₃H₂,
- a C(O)CH₂C(O)Alk group, a C(O)CH(Alk)C(O)Alk group, a C(O)CH₂C(O)H group, a C(O)CH(Alk)C(O)H; wherein Alk is a linear or branched alkyl group comprising from 1 to 30 carbon atoms, preferably from 1 to 5 carbon atoms;
- an aryl substituted by a COOH or two OH groups in ortho position one relative to the other, a PO₃H₂ group, a C(O)CH₂C(O)Alk group, a C(O)CH(Alk)C(O)Alk group, a C(O)CH₂C(O)H group, a C(O)CH(Alk)C(O)H; wherein Alk is a linear or branched alkyl group comprising from 1 to 30 carbon atoms, preferably from 1 to 5 carbon atoms.

4. Compound according to claim 1, in which :
each R¹ and R² are identical or different and represent:
- a hydrogen atom;
- an aliphatic chain, linear or branched, comprising from 1 to 30 carbon atoms;
- a group of formula -(L¹)ₙ-A-COR³; -(L¹)ₙ-A-PO₃H₂; or -A-(R⁵)_{q};
with the proviso that at least one of R¹ and/or of R² is different from H and at least one of R¹ and/or R²-(L¹)ₙ-A-COR³; -(L¹)ₙ-A-PO₃H₂; or -A-(R⁵)_{q};
A represents a cycloalkyl, heterocycloalkyl, aryl or heteroaryl group, monocyclic or polycyclic, each optionally mono- or poly-substituted for example by a linear or branched alkyl comprising from 1 to 30 carbon atoms and/or a group OR⁴; or an aliphatic chain, linear or branched, comprising from 1 to 30 carbon atoms;
R³ represents hydrogen, OR⁴, a linear or branched alkyl comprising from 1 to 30 carbon atoms and optionally substituted by at least one group (=O) and/or OR⁴;
R⁴ represents H, a linear or branched alkyl comprising from 1 to 30 carbon atoms;
q is an integer and represents from 1 to 5, for example 1 or 2;
R⁵, identical or different represents:
- OR⁴;
- an alkyl chain, linear or branched comprising from 1 to 30 carbon atoms optionally substituted by at least C(=O); OR⁴ group, COR³, and/or PO₃H₂;
- aryl or heteroaryl optionally mono or polysubstituted by:
○ a linear or branched alkyl comprising from 1 to 30 carbon atoms optionally substituted by at least one group (=O); OR⁴ group, a group PO₃H₂, and/or a group COR³;
○ a group OR⁴;
○ a group PO₃H₂; and/or
○ a group COR³.

5. Compound according to claim 4 wherein each R¹ and R² are identical or different and represent:
- a hydrogen atom;
- an aliphatic chain, linear or branched, comprising from 1 to 30 carbon atoms;
- a group of formula -(L¹)ₙ-A-COOR⁴; -(L¹)ₙ-A-C(O)CH₂C(O)Alk , -(L¹)ₙ-A-C(O)CH(Alk)C(O)Alk, -(L¹)ₙ-A-C(O)CH₂C(O)H, -(L¹)ₙ-A-C(O)CH(Alk)C(O)H ; -(L¹)ₙ-A-PO₃H₂; -A-aryl, the aryl being substituted by two OH group in ortho position one relative to the other and/or one CO group and one OH group in ortho position one relative to the other;
with the proviso that at least one of R¹ and/or of R² is different from H and at least one of R¹ and/or R² is a group of formula -(L¹)ₙ-A-COOR⁴; -(L¹)ₙ-A-C(O)CH₂C(O)Alk , -(L¹)ₙ-A-C(O)CH(Alk)C(O)Alk, -(L¹)ₙ-A-C(O)CH₂C(O)H, -(L¹)ₙ-A-C(O)CH(Alk)C(O)H; -(L¹)ₙ-A-PO₃H₂; -A-aryl, the aryl being substituted by two OH groups in ortho position one relative to the other and/or one CO group and one OH group in ortho position one relative to the other;
n and L¹ being as defined in claim 1;
R⁴ represents H, a linear or branched alkyl comprising from 1 to 30 carbon atoms;
Alk represents a linear or branched alkyl comprising from 1 to 30 carbon atoms;
A represents an aryl or heteroaryl group, monocyclic or polycyclic, preferably aryl, for example phenyl, each optionally mono- or poly-substituted for example by a linear or branched alkyl comprising from 1 to 30 carbon atoms and/or a group OR⁴.

6. Compound according to claim 4 or 5, wherein each R¹ and R² are identical or different and represent:
- a hydrogen atom;
- an aliphatic chain, linear or branched, comprising from 1 to 30 carbon atoms;
- a group of formula -(L¹)ₙ-A¹-COOH wherein A¹ represents a cycloalkyl, heterocycle, aryl or heteroaryl group, preferably aryl, preferably phenyl, monocyclic or polycyclic, the monocycle or polycycle being optionally mono- or polysubstituted by for example by a linear or branched alkyl comprising from 1 to 30 carbon atoms or a group OR⁴;
with the proviso that at least one of R¹ and/or R² is different from H and at least one of R¹ and/or R² is a group of formula -(L¹)ₙ-A¹-COOH.

7. Compound according to claim 4 or 5, wherein each R¹ and R² are identical or different and represent:
- a hydrogen atom;
- an aliphatic chain, linear or branched, comprising from 1 to 30 carbon atoms;
- a group of formula alkyl-COOH, wherein the alkyl is linear or branched and comprised from 1 to 30 carbon atoms, preferably from 1 to 10 carbon atoms, for example from 1 to 5 carbon atoms,
with the proviso that at least one of R¹ and/or R² is different from H and at least one of R¹ and/or R² is of formula alkyl-COOH, wherein the alkyl is linear or branched and comprised from 1 to 30 carbon atoms, preferably from 1 to 10 carbon atoms, for example from 1 to 5 carbon atoms.

8. Compound according to anyone of claims 1 to 7, in which R² represents H, with the proviso that at least one of R¹ is different from H.

9. Compound according to anyone of claims 1 to 8, of formula (Ia), (Ib), (Ic), (Id), (Ic'), (Id'), (Ie) to (Ii): 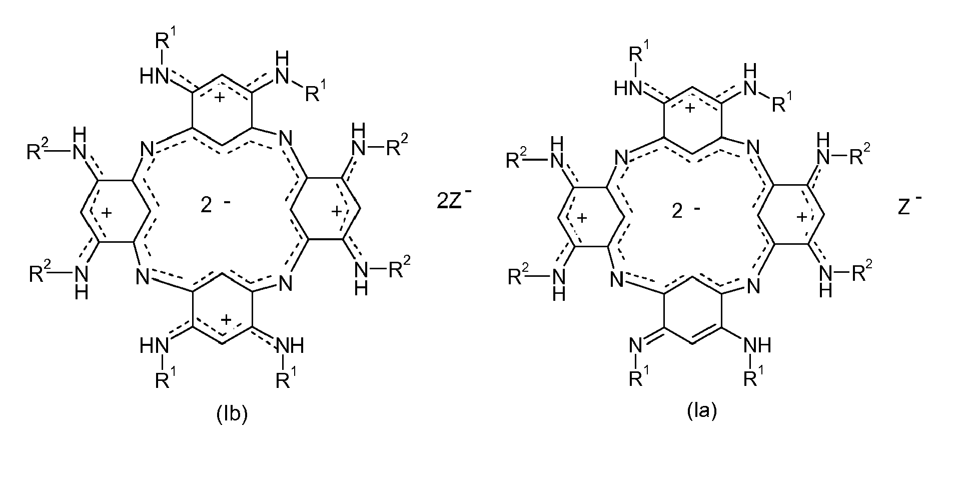 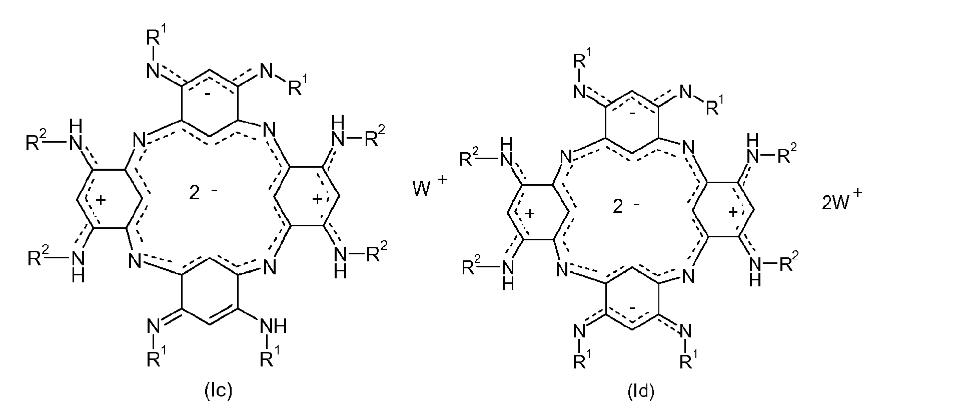 Or 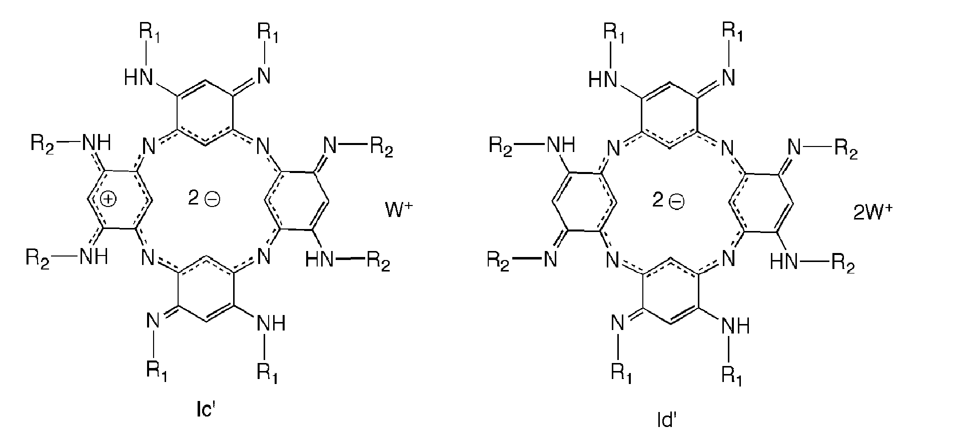 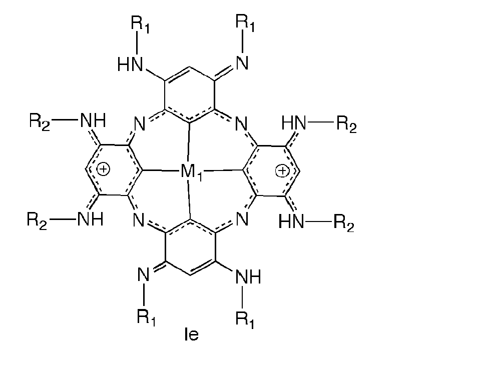 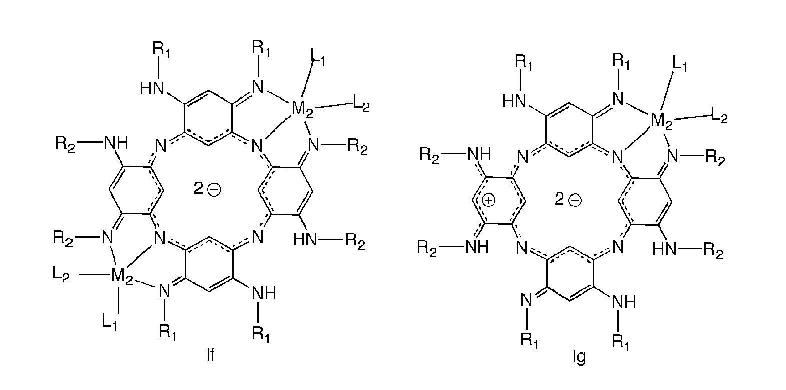 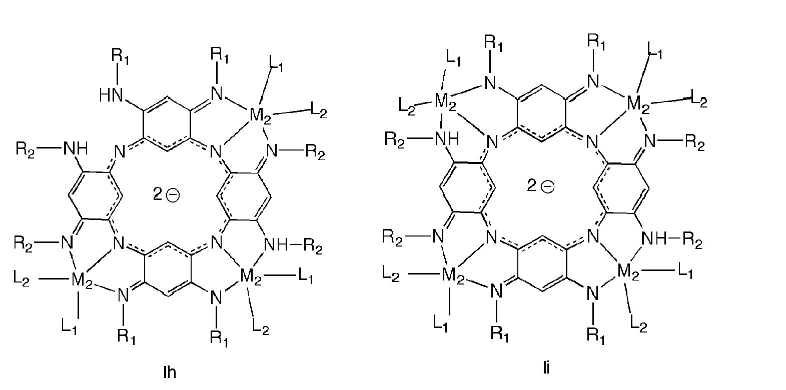 wherein:
R₁ and R₂ are defined as in claims 1 to 8,
M₁ is chosen among the transition metals having a degree of oxidation of 4 and M₂ is chosen among the transition metals having a degree of oxidation of 2 or lanthanides;
L₁ and L₂, identical or different, is a ligand which can give 1 covalent bond or 2 electrons, and/or may be optionally bound together.
Z represents an anion, preferably from an halogen atom, BF₄, PF₆, ClO₄, CF₃COO, or CF₃COO and W represents Na, Li, NEt₃.

10. Compound according to anyone of claims 1 to 9, having a Eg gap comprised between 1 and 4 eV.

11. Process for the preparation of compounds according to anyone of claims 1 to 9 chosen among:
- A process (P1) comprising the following steps:
a1) reacting a 1,5-Q₂-2,4-dinitrobenzene with 0,5 equivalent of tetraaminobenzene in the presence of a base, Q, identical or different being a leaving group;
b1) reacting the compound obtained in step a1) with one equivalent of a compound derived from tetraaminobenzene in which at least one and at most two of NH₂ group is substituted with a group R¹ or R² different from H, in the presence of a base;
c1) reduction of the compound obtained in step b1) in the presence of a reducing agent which under air is converted into compound of formula (I); or
- A process (P2) comprising the following steps:
a2) reacting a 1,5-Q₂-2,4-dinitrobenzene with 1 equivalent of a compound derived from tetraaminobenzene in which at least one and at most two of NH₂ group is substituted with a group R¹ or R² different from H, in the presence of a base, Q, identical or different being a leaving group;
b2) reduction of the compound obtained in step a2) in the presence of a reducing agent which under air is converted into compound of formula (I); or
- A process (P3) comprising the following steps:
a3) reacting a 1,5-Q₂-2,4-dinitrobenzene with 0,5 equivalent of a compound derived from tetraaminobenzene in which at least one and at most two of NH₂ group is substituted with a first group R¹ or R² different from H, in the presence of a base, Q, identical or different being a leaving group;
b3) reacting the compound obtained in step a3) with one equivalent of a compound derived from tetraaminobenzene in which at least one and at most two of NH₂ group is substituted with a second group R¹ or R² different from H and different from the one of step a3), in the presence of a base;
c3) reduction of the compound obtained in step b3) in the presence of a reducing agent which under air is converted into compound of formula (I).

12. Process for the preparation of compound (Ie) to (Ii) according to claim 9 comprising the reaction of a compound of formula (I) with at least one mole of an acid or with at least one mole of a base.

13. Use of compound of formula (I) according to anyone of claims 1 to 10 in solar cells, such as organic solar cells and particularly as dye in dye-sensitized solar cells (Grätzel cells).

14. A complex having as ligand a compound of formula (I) according to anyone of claims 1 to 10.

15. A solar cell, such as organic solar cell and preferably dye-sensitized solar cell (Grätzel cell), comprising a compound according to anyone of claims 1 to 10.
